Europäisches Patentamt

European Patent Office

Office européen des brevets

(19)

(11) Publication number: 0 372 911 A2

## EUROPEAN PATENT APPLICATION

(21) Application number: 89312678.9

(51) Int. Cl.⁵: C12M 1/40

(22) Date of filing: 05.12.89

(30) Priority: 05.12.88 CS 7992/88

(43) Date of publication of application:
13.06.90 Bulletin 90/24

(84) Designated Contracting States:
AT BE CH DE FR GB IT LI LU NL SE

(71) Applicant: CHIRANA Vyzkumny ustav
zdravotnicke techniky koncernova ucelova
organizace
No 3 Kamenice
Brno(CS)

(72) Inventor: Krejci, Jan
No. 593 Jiraskova
CS-Tisnov(CS)
Inventor: Macholan, Lumir
No.29, Sumavska
CS-Brno(CS)

(74) Representative: Ablewhite, Alan James
MARKS & CLERK 57/60 Lincoln's Inn Fields
London WC2A 3LS(GB)

(54) Recoverable enzyme sensor with replacement membrane system.

(57) A recoverable enzyme sensor is created by a body 3a of coroundum ceramics in the shape of a cylinder being terminated with a truncated taper 12 in which a reservoir 10 is created in the shape of cylinder with the base of circular ring inside of which both a current electrode 5 made of porous metal and electrolyte e.g. potassium chloride, are arranged; in the cross-section parallel with the basis line the first ceramics layer 17 of the reservoir 10 has the shape of cylinder in which a reference electrode 4 is arranged and further, the body 3 is covered with a covering membrane 9 and connected with the cover 2 created by the holder 1 equipped with at least one opening 14 in which there are arranged at least one enzyme membrane 8, at least one protecting membrane 11, and at least one control membrane 20.

FIG. 2

## Recoverable Enzyme Sensor With Replacement Membrane System

The invention relates to a recoverable enzyme sensor with a replaceable membrane system.

The goal of the present invention is to find a constructional design of the enzyme sensor enabling the replacement of the enzyme in the reacting membrane so that glucose, lactate or other substances may be either used for a long time span, or easy recovered or replaced.

The enzyme sensor consists of two basic parts:

1. Membrane system
2. Electrode system

The different parts can be defined as follows:

The electrode system - a set of two electrodes, at least, being assembled as a single operating unit being provided with interconnection wires, input cable and output connector to connect a processing unit.

The electrode - this is part of electrode system containing an electrically conductive and (solution and/or melt and/or solid state) electrolyte.

The membrane system - this generally comprises at least one or a set of two electrodes, which lie one on the other differing from each other by their response characteristics and chemical composition and which control the entry of the analyte to be measured to the electrode system in which the selective biochemical reactions are carried out to determine the analyte of interest.

Some examples of electrode arrangements which have been used for electrochemical and analytical assays have been reviewed (e.g. Linek V., Vacek V., Sinkule J. in Dissolved Oxygen Probes). The electrode system can be prepared using microelectronic procedures. The properties of electrodes and electrode systems have been discussed theoretically in detail (e.g. Lonek V., Vacek V., Sinkule J.: Dissolved Oxygen Probes in W.C. Robinson (editor) Comprehensive Biotechnology, Vol. 3, Chapter 22, Pergamon Press, Oxford 1984; Koryta J., Stuklik K. Ions selectivity electrodes, Academia, Prague, 1984; Vasely J., Weiss D., Stuklik K. Assays using ion-selective electrodes, SNTL Prague, 1979; Dvorak J., Koryta J.: Electrochemistry Prentice Hall, Inc. Engelwood Cliffs 1987).

Membrane systems have been described theoretically in detail. However, some membrane systems are lacking in stability when assaying the analytes of interest or they show low selectivity. In some electrode systems, the immobilization of the enzyme is troublesome. A suitable example could be the membrane system of the glucose analyser manufactured by Yellow Springs Instrument which consists of a polycarbonate membrane, immobilized enzyme and cellulose membrances with pore diameters of 0.6 nm and 30 nm, respectively, the enzyme being immobilized in between both membranes.

The basic operation principles of the membrane system (Baum G., Weetall H.H.: Electrochemical applications of oxyreductases. Methods in enzymology, LVI part 6, 1979).

1. It limits the biochemical-electrochemical process in the electrode system by diffusion;
2. It lowers the effects associated with stirring;
3. It ensures that the reaction area has a well defined geometrical arrangement;
4. It ensures the transport of analytes to the electrode system;
5. It influences both the response rate and sensor linearity carrier of immobilized enzymes;
6. It secures a high enzyme activity in the well defined area;
7. It prevents unwanted substances from entering the electrode system.

The construction of the electrode system can be explained on the electrode system construction being used for the $O_2$ detection (Clark cell).

Material of cathode: the most suitable material for the cathode is silver. However, its main disadvantage is in that it cannot be sealed in the glass which proved to be necessary for an insulation resistance of an adequately high value. Further materials being used for cathode manufacture are gold, platinum or carbon.

Electrodes of carbon which are specially treated have the advantage there are no problems observed with biocompatibility. The purity of the material, the treatment and tooling all considerably influence the shape of the polarographic curve and, thus, the operation capability of the sensor.

In general, it can be stated that the lower the purity of the materials used for the manufacture of the cathode, the lower will be the diffusion current, the narrower the flat course on the polarographic curve and the lower the polarographic curve being parallel with the voltage axis in the area of the limiting diffuse current. These circumstances influence the operability of the electrodes system considerably and they may influence the linearity of its response characteristic. Similarly, the polarographic curve shape could be influenced by the treatment of the cathode surface. It has been proven that the optimum roughness of the cathode surface is 1 μm. Further, the shape of the polarographic curve may be influenced by the both size and electrode construction. The purity of the material used influences the way of tooling and interconnecting the wires to the material of cathode, because during these operations the surface of cathode might be contaminated. Further, some specific requirements should be considered for other metals. Gold and silver are not the stable ones. Silver may be easy poisoned with trace amount of hydrogen sulfide and darken. Platinum has properties which are strongly dependent on the surface conditions and the fabrication history (polishing, grinding). In acid surroundings, the electrode reaction velocity is lowered on platinum. The surface of a carbon electrode should be activated using the proper procedure. The dimensions of all cathodes should not be lower than 0.1 mm.

Anode - its dimensions should not be less than $10^3$ times greater than those of the cathode. It is required that the anode shall not be the polarizable one. Conventionally, argentchloride electrodes have been used. Palladium usage in conjunction with enzyme membrances has been known, too. This arrangement improves the probe stability. The minimum palladium purity is 99%

Electrolyte - the prior art shows that the electrolyte should be available in adequate volume to ensure long term stability and the changes due to chemical reactions should be ignorable. As the electrolyte, potassium chloride is mostly used. However, it should be saturated (it causes unwanted silver deposition on the cathode causing the properties of the probe to change and the residual current to increase). A concentration of 0.75 M has been recommended. In some cases a solution of potassium hydroxide can be used instead, but it has worse properties than those of potassium chloride. As buffers, potassium hydrogen carbonate, sodium hydrogen carbonate, a composition of potassium carbonate and potassium hydrogen carbonate, and acetate buffer can be used. To prevent changes of concentration of electrolyte from occurring because of water diffusion, sodium nitrate is added. For a prolonged utility time span some modified electrolyte systems have been introduced, for example ion-exchange membranes, electrolyte soaked in the membrane substrate, or electrolyte contained in a gel of suitable composition, and mediators.

Body, electrode attachments, geometrical arrangement: all materials used should have high resistivity and they should be inert to salt solutions. The design arrangement should be such that between electrodes the resistance is in excess of $10^9$ ohm, at least. Experimentally, it has been found the cathode should free touch the ideal membrane. The geometrical probe arrangement as a whole should be such so that the inlet analytes, e.g. oxygen, may diffuse perpendicularly to the plane of cathode i.e. that the concentration gradient may be in the direction of the normal to the surface of the measuring electrode (cathode) so that the so-called side diffusion causing slow sensitivity decrease, hysteresis of probe and change of dynamic response may be prevented. If it is the case the before-mentioned requirements have been met, there is an output signal from the electrode system covered with the ideal membrane system permeable to oxygen proportionally to the partial pressure of the oxygen.

Non-linearities can be caused by:

a) a successive chemisorption of oxygen on the cathode surface. It can be removed e.g. by an impulse of current of suitable duration and intensity;

b) an absorption of impurities, both chemical and physical. The chemisorption can be removed by the same procedure described in the preceeding point;

c) a high resistance of the argentchloride anode; the successive cumulation of silver chloride on the anode can change its resistance causing the response characteristic of the electrode system to change;

d) an increasing size of cathode; while the area of cathode is of adequate size there is possibly no homogenous interaction between the cathode and the membrane system to result in nonlinearity. The size of the active area of membrane taking part on the reaction changes, as well;

e) an interaction with the membrane system; the membrane system not being designed carefully and being in direct contact with the electrode could also cause non-linearities.

The stability of the electrode system: the stability of the electrode system can be divided in short term stability and long term stability. The nonstabilities can be divided into:


Short term non-stability:


a) This can be caused by reduction of the surface oxides of metals. This is the original probe non-stability, disappearing in some minutes to 10 hours. This non-stability can be of importance during mass production of probes. To prevent it from occurring, the probe should be maintained under a constant polarization voltage as it is the Miles Laboratories Inc. process.

b) It can be caused by bromine, dinitrogen oxide or carbon dioxide.

c) It can be caused by contamination of the platinum electrode with ions of other metals. This kind of non-stability goes to the long term non-stability category, as a rule. The possibility of this non-stability occurring requires a sophisticated technology of treatment and high purity.

d) It can be caused by an interaction of the membrane system with the electrode system. Non-stability of this kind can, e.g., be caused by a movement of the covering membrane. Its elimination increases the construction requirements on the interface between the electrode system and the membrane system.

e) It can be caused by a movement of electrolyte between the cathode and the membrane system. For this kind of non-stability the same comments apply as in the preceeding point.

f) Further, it can be caused by a pH value change. As follows from the known state-of-art, while the probe is operating the pH value can change. Thus, the polarographic curve is changed which could result in a sensitivity change acting as a short term non-stability or like a long term non-stability (depending on the shape of the polarographic curve and polarization voltage on which the electrode system operates). To remove this non-stability source, the volume of electrolyte must be adequate with the preference of a buffer electrolyte.

Long term non-stability:

a) This can be caused by slow side-reactions; this type of non-stability can be caused by a chemisorption, a physical adsorption of electrolyte additives or by interfering reactions between the cathode and impurities in electrolyte. This kind of non-stability can be prevented by using very highly pure chemicals and polishing the cathode before operation.

b) It can be caused by changes of anode state; through slow dissolving of the anode and its silver chloride plating, the properties of the anode can be changed. The main influences are a change in the active area, an increase of resistance because of too thick a layer of silver chloride, an increase of chloride ions in the electrolyte, consumption of the anode and elimination of silver on the cathode. These types of non-stability can be prevented from occurring with a large area of anode, high purity of the anode, suitable electrolyte concentration and the correct technology in the preparation.

c) Further, it can be caused by wrong chlorination of the argentochloride anode.

d) Further it can be caused by adsorption of small particles on the anode. Again, to prevent this, a suitable sequence of technological operation, and purity while preparing, should be observed.

e) Finally, it can be caused by a leakage between electrodes. During the entire time span for which the electrode system is desired to operate reliability, the resistance between the electrodes must be maintained in excess of $10^9$ ohms. In some sealing cements conductive channels can form, short circuiting the electrodes, and a long term non-stability appears.

The residual current; in the ideal case in which none of the beforementioned problems is observed, there is no electrical current flowing between the electrodes. Because of manufacturing imperfections, impurities, leakages and further events, there is in fact an electric current flowing between electrodes even if there is no oxygen present, this current being called the residual current. In a well constructed electrode system, the magnitude of the residual current can be neglected (1-3%). In cases in which the residual current is high, and at the same time stable, it can be electrically compensated. Should it be the case the residual current is non-stable and, at the same time, high the probe cannot be used at all.

The sources of residual current are as follows:

1. Reduction of electrochemically active impurities on the cathode, in the electrolyte, or coming from the surroundings. It can be remedied by usage of very high purity materials and materials possessing a very high chemical stability.

2. Hydrogen elimination in electrolyte solution when the polarization voltage is high. To remove it, the position of the polarization voltage on the polarization curve could be changed.

3. Insufficient insulation between electrodes. The same comments apply as for the long term non-stabilities. The material recommended for use in insulating the cathode is glass or polyfluoroethylene. The disadvantage of glass is in that surface currents occur in the construction of the electrode system and also a nonreliable glass state on the interface glass-seal used for design of the electrode systems.

4. Oxygen dissolved in electrolyte. The residual current can be caused by oxygen detection in the electrolyte, to which it can become added by diffusion through the walls of the electrode system. Because most plastics have good diffusion properties for oxygen, this may restrict utilization of plastics materials for the construction of the body.

The dynamic response characteristics of the electrode system: The dynamic response characteristics are uniquely determined by the transition characteristic of the electrode system. As stated hereinbefore, the optimum performance of the electrode system of the oxygen probe requires a very thin layer of electrolyte. Because of this, the electrolyte effect on the dyanmic performance can be neglected.

Empirically, the following relationship has been observed

4

$$\tau_{95} \propto 1 \text{ if } 1 \ll d \quad \text{(I)}$$

where $\tau_{95}$ is the time span during which the probe response reaches 95% of its maximum value, d is the diameter of the cathode and 1 is the thickness of the electrolyte layer.

The condition (I), however, counteracts the requirement for quick removal of hydroxide ions which could cause significant changes in the pH value in the electrolyte layer and, thus, in its final result, could change the performance characteristic under dynamic conditions without any dependence on the partial oxygen pressure. Experimentally, cathodes having diameters ranging from 1 to 10 micrometers have been evaluated. With such small dimensions, the analyte concentrations in the vicinity of the cathode can be quickly equalized by diffusion; thus these electrodes require no stirring. The causes of changes in the probe dynamic response characteristic can be as follows:

1. Change of the geometrical arrangment of the electrode (cathode) and the ideal membrane resulting in the side diffusion of oxygen.

2. Interaction with the membrane system

3. Side diffusion from the electrolyte

4. Insufficient velocity of hydrogen peroxide decomposition.

The dynamic response characteristics of the sensor in the ideal case show no hysteresis in real sensors, however, hysteresis often appears i.e. the resulting signal is dependent on the history of the electrode polarization.

It can be caused by the following effects:

1. It can be caused either by size, shape or overall arrangement of the electrolyte reservoir;

2. Further, it can be caused by the size of the electrolyte film;

3. Further, it can be caused by electrochemical process (adsorption);

4. Further, it can be caused by mechanical stresses in membranes of the membrane system.

The most significant effect of these is the interaction with the membrane system on the dynamic behaviour of the probe.

Temperature effects: The temperature considerably affects the behaviour of the oxygen probe. Especially, the temperature influences the sensitivity and the response time of the probe. The response of the probe is given both by electrochemical reaction on the cathode and by oxygen transportation. The velocity of these events depends exponentially on the temperature. The machanism of both events can be classified by means of activation energies. It has been known that the average change in probe parameters is 1-6% per 1 degree.

For an accurate measurement, a temperature stability to 0.1 degree is necessary. Empirically, the best temperature behaviour has been observed when polytetrafluoroethylene is used as the covering membrane.

Elimination of temperature effects:

1. Thermostating the probe

2. Interconnecting the probe with a thermistor or another temperature pick-up, for compensation of results.

Pressure effects:

From the known facts it follows that a pressure change causes the shape and the thickness of the electrolyte layer to change, resulting in changes of related characteristics. To some extent the pressure is thus responsible for sensitivity, dynamic characteristic and stability.

It should be noted that to cover sensors, a membrane system having a thickness of approximately 10 micrometers has been used. (The small thickness of the membrane system is necessitated by the quick response requirement).

The pressure changes ranging in Pa units could have a considerable influence on the measurement.

Discussion of the construction of the membrane system. The membrane system secures the proper analytical reaction. In a plurality of cases, it consists of a covering membrane separating enzyme membranes in which the analytical reaction take place at the electrode system. The enzyme membranes are separated by controlling membranes ensuring that an adequate flux of analytes to the different components of the enzyme membranes is available. Overall characteristics of the membrane system:

For an operable sensor, it should be ensured that the parameters of the membrane system may be constant (thickness of different membranes, their diffusivity, mechanical properties etc).

The membrane system can effect the linearity of the sensor. The main causes of the nonlinearity can be as follows:

a) Nonhomogenous homing of the membrane system with the electrode system; nonequal thickness of the different membranes of the membrane system (while the significance decreases with the distance from the cathode).

b) Mechanical stress in the membrane system: by trial, it has been proved that the membrane system should be "free applied" to the electrode system.

c) Nonlinear dependence of the membrane permeability on the concentration: the diffusivity changes (permeability) with the stress.

Nonlinearities of the beforementioned kinds can be avoided by suitable application and fixation of the membrane system.

Short term nonstability can be caused by one or both of the following causes:

a) Wrong attachment of the membrane system and its movement towards the electrode system.

b) Hydration of membranes resulting in parameter changes (diffusivity, dimensions).

Long term nonstability can be caused by one or more of the following causes:

a) Degradation of the membrane system: slow decrease in diffusivities, changes in the membrane stress; washing out of enzymes and denaturating the enzyme.

b) Water diffusion or ion diffusion into the electrode system resulting in electrolyte concentration changes. Disadvantageous properties of some membrances.

c) Diffusion of substances which are able to poison the cathode in the electrode system.

Sensitivity: In the ideal case it can be determined by the "diffusive" resistance of the membrane system.

Dynamic response characteristic of the sensor.

The phenomena relating to the membrane system and most significantly influencing the dynamic resposne characteristic include the following:

a) irregular contact of the surface of the membrane system with the electrode system

b) mechanical stresses in the membrane system

c) thin-layer fluid film on the surface of the membrane system

d) deposition of substances from the solution to be measured onto the surface of the membrane system.

The points c) and d) can cause serious failures during measurement using the electrochemical sensor.

Temeprature influence:

The influence of the temperature on the membrane system operation is very serious because both the transient events and the chemical reactions taking part are temperature dependent by an exponential relationship.

The description of the both events can be carried out conventionally by means of the activation energy. The (upper) limit of the temperature dependence can be determined. For any membrane and any chemical reaction approximately 2% change per degree can be observed while the result being given changes with the temperature.

Pressure effects:

The effect of pressure results in dimension changes in the membrane system and occurence of stresses in the membrane system. The overall change of the membrane system position results in a change in the electrolyte layer thickness. This effect has been discusssed in the case of the electrolyte system. The stresses in the membrane system can cause a change of its diffuse response characteristics and in this way they can effect its sensitivity. If relaxation of the membrane system materials is the case, this can result in long term non-stability.

A number of the present electrode types have been so designed that some theoretical requirements described in the beforementioned description might be met.

a) Macroelectrodes. To detect enzymatic reactions consuming oxygen, in the most cases, recently oxygen electrodes have been used which are provided with an attached enzymatic membrane. Because of the fact that the oxygen electrode has one measuring electrode only, the measurement must be carried out in surroundings having a defined partial oxygen pressure. Anodes have been made of argent chloride. The electrode as a complete assembly is very massive (some effort has been observed to standardize the

6

diameter to be 12 mm). The electrolyte can be easily replaced. A disadvantage of these types of electrode is their relatively high price. Another disadvantage has been the unfeasibility of miniaturize them while manufacturing. Another disadvantage is in that in many types of electrode the membrane support is not accurately defined, so that the output signal may be dependent on pressure from the electrode. Sensors of this type can be used in specialized laboratories only, and no unskilled personnel can be allowed to operate them, (e.g. the glucose sensor in the personal glucose meter). These disadvantages are remedied with the present recoverable sensor according to the present invention.

b) Planar microsensors. These have been fabricated using microtechnological procedures on a suitable substrate. This approach has resulted in a low price at high number manufacturing runs. The utilization of microelectronic procedures enables the creation of miniature electrode systems containing multiple electrodes and the pick-ups for temperature measurement, if required for the temperature compensation. These systems are being developed, now, and their disadvantages cannot yet be completely evaluated. From the initial results, however, it follows that the attachment means of memebranes and the presence of electrolyte seem to be the questions which have not yet been solved from the point of view of their long term use. These disadvantages are remedied by the recoverable sensor of the present invention, in that it utilises a massive (in comparison with the sizes of the other electrodes) porous anode, the pores of which serve as the electrolyte reservoir.

c) Needle electrodes. Examples of needle electrodes have been described in the following literature articles: Schichiri, M.; Kawamori R.; Glycaemic Control in Pancreatomized Dogs with a wearable artificial Endocrine Pancreas. Diabetologia, 1983, 24, 179-184. Schichiri, M.; Kawamori, R.: Wearable artificial endocrine pancreas with needle-type glucose sensor. The Lancet, November 20, 1982, pp 1129-1131. Schichiri, M.; Kawamori, R.: Long-term Glycemic Control with a Portable system for Insulin Delivery, edited by P. Brunetti et al. Raven Press, New York, 1983.

The needle electrode reviewed in the abovementioned articles has been manufactured by sealing a small platinum wire into a glass cylinder inserted into a silver covering serving, at the same time, as the reference electrode. The front part of the sealed sylinder is polished and on the polished surface an enzyme is immobilized being covered with a cellulose membrane containing heparin. The complete point of the needle electrode is covered with a polyurethane membrane.

Ikeda, S.; Kimura, M.; Ito, K.: Needle-type glucose sensors based on oxygen electrodes with a new concept extended abstracts, Electrochemia Society meeting 18-23.10.1987.

The needle electrode reviewed in this article consists of two self-contained oxygen electrodes arranged in series, of which one is coated with an enzyme.

The disadvantage of needle electrodes consists in that the electrolyte has not been defined and also, generally, that the surface area is not well defined, and the conditions for satisfactory performance of the electrochemical sensor are not met satisfactorily.

The present disadvantage described in the paragraphs a), b) and c) are remedied by the recoverable enzyme sensor of the present invention, i.e. a recoverable enzyme sensor with an interchangeable membrane system characterised in that it comprises a ceramic body 3 having disposed therein a current electrode 5 in the form of a porous metal insert occupying a reservoir 10 for electrolyte, a reference electrode 4 and at least one measuring electrode 6, said electrodes being arranged so that they each have a contact surface at a common surface 13 of the ceramic body such that the contact surface of the current electrode 5 surrounds that of the reference electrode 4 and the contact surface of each measuring electrode 6 is outside the surrounding electrode 5, said common surface 13 being provided with a permeable membrane cover 9, the body 3 being provided with a removable cover 2 to hold an exchangeable enzyme membrane 8 in contact with said common surface 13 covered by the membrane cover 9.

The ceramic body is preferably in the shape of cylinder, the body being terminated with a truncated taper in which there is a reservoir created having the shape of an annular cylinder inside which thre are disposed both the porous metal current electrode and the electrolyte, e.g. potassium chloride, while in the cross-section perpendicular to the axis the ceramic body comprises an inner ceramic layer shaped as a circular ring in which there is arranged one measuring electrode, at least, and an outer ceramic layer has a shape (in section) of a co-axial ring in which there is the reference electrode arranged and, further, the body is both covered with a covering membrane and connected with the housing of the probe to which the replaceable membrane system is attached by a holder equipped with an aperture, in which there are arranged at least one enzyme membrane, at least one protective membrane and at least one control membrane.

The recoverable enzyme sensor is further characterised by:
the body being made of silicone corundum ceramics having a resistivity of at least value of $10^{12}$ ohm.m;
the first ceramics layer of the reservoir being provided with at least one projection arranged on the inner part of the circular ring, a measuring electrode being arranged in the projection;

the cover of the sensor being made of elastomer or tough plastics, the mechanical properties of which, e.g. the hardness, elasticity and toughness, secure airtight connection of the covering membrane with the taper of the body of the electrode system;

the holder being made of plastics and arranged for disassemblable connection with the cover of the sensor;

the holder being provided with an arresting projection;

orifices in which the membrane system is arranged, located over the different measureing electrodes;

the holder having a circular shape and being provided with orifices in which there are arranged enzymatic membranes equipped with the appropriated enzyme e.g. with the glucose oxidase, and further, with a control membrane which has a pore size corresponding with the sizes of the molecules to be measured and with a protective membrane having a negligible diffuse resistivity;

the orifices being provided with meshes;

the measuring electrode being of a metal selected from platinum, silver, gold, tantalum, molybdenum, irridium, osmium and tungsten;

the front surface of the body taper being provided with electrode structures created by covering with other metals e.g. gold, silver or platinum or its alloys, in ratio rating from 0.1 to 99.9% by weight, respectively;

the front surface of the body taper being polished and having a roughness ranging from 0.1 to 50 $\mu$m;

the front surface of the body taper projecting over the measuring electrode by 1 to 10 $\mu$m;

the measuring electrode being made of an alloy of gold with platinum in the ratio range 0.0 to 99.9% by weight and platinum with gold ratio ranging 0.1 to 99.9% by weight;

the measuring electrode being made of non-metal material, e.g. carbon;

the reference electrode being made of metal, e.g. platinum, and its front surface being covered with a layer of silver or palladium;

the current electrode being made of silver having purity of at least 97%;

the electrolyte being contained in the porous current electrode;

the body, cover and holder all being mutually connected in a disassemblable way;

the current electrode being made by sintering silver powder in a hydrogen atmosphere at a temperature ranging from 800 to 900 ° C;

the current electrode being treated by chlorination in gaseous chlorine.

The new and improved effect can be seen not only in the fact that the sensor complies with the main requirements of the present state-of-art (see the introduction), but in addition, that its constructions design enables, further:

Making the cathode of silver, either by silver paste (amalgam) deposition with subsequent burning, or by deposition of silver using microelectronics procedures at the outlets of platinum electrode sites. This procedure assumes a corundum ceramics is used, to which the silver has a very good adhesivity. A similar advantage can be obtained with gold, as well.

If platinum is the case, the platinum cathode can be used directly in the corundum ceramics.

When platinum electrodes are made of calibrated wires, a high symmetry of the electrode surface can be attained.

Because the electrodes are closely arranged one to another, all will be influenced in the same manner during treatment (e.g. polishing) and the resulting sensor will possess symetrical electrodes. Because the measurement is carried out differentially, the effect of the manufacturing history is eliminated in platinum electrodes.

The anode of the sensor in accordance with the present invention is made by sintering the silver powder in the hydrogen atmosphere with subsequent chlorination in gaseous chlorine.

This process ensures that the surface area of the anode is by some orders higher than that of the cathode, with a very small volume. The sintering in hydrogen surroundings ensures complete cleaning of silver and the subsequent chlorination operation makes a homogenous compact layer of silver chloride.

The anode is porous. By this fact, a storage reservoir for electrolyte in minute volume is created at the same time. By this arrangement an electrolyte storage reservoir of minute volume can be made in a relatively small sensor.

The body of the sensor is made of corundum ceramics having a high resistivity and being inert to salt solutions so that a resistance between electrodes in excess of $10^{10}$ ohms can be maintained while the electrolyte present, for a long time span. When corundum ceramics is used a very homogenous connection of the cathode metal (e.g. platinum) with the ceramics (helium seal) can be attained. By this means it is ensured that the electrode reaction takes place only on the front surface of the sensor and no side reactions occur at a longer distance from the membrane system. In this way hysteresis is prevented, there are no dynamic performance characteristic changes and no sensitivity impairment.

By the construction of the sensor in compliance with the present invention, some sources of non-linearities are limited as well. Because of the same operating procedure during the treatment of the front part of sensor, and the differentiated way during measurement both chemisorbtion and impurity - adsorbtions (both chemical and physical) have less effect. By the chlorination process in gaseous chlorine and by the large surface area of the anode (it is porous) the effect of non-linearity caused by some non-homogenous silver chloride distribution is limited, as well. Because of the fact that the electrodes are burned in the corundum ceramics successful increasing of the cathode size is not possible and the associated non-linearity is limited, as well. The contact with the membrane system is ensured by mechanical means. Because the measurement is carried out differentially on symmetrical cathodes, the effect of non-linearity can be partially eliminated, as these effects can be compensated for in the differential measurement procedure.

The sensor according to the present invention limits some other effects of long term non-stability. It limits some non-stabilities caused by side reactions by means of the way the body is formed of inert sintered ceramics which can be compactly connected with the electrodes.

Further, it limits the long term non-stabilities caused by changes of anode parameters, by the fact that the anode used has a large surface area. Further, the long term non-stabilities caused by wrong chlorination processes, by the means of the fact that the anode is completely cleaned out while sintering in the hydrogen atmosphere and the next chlorination in gaseous chlorine makes a homogenous layer of the silver chloride, are supressed, as well. Further, the long term non-stabilities caused by conductive bridge can be limited by the way that the body of the sensor is made of inert sintered corundum ceramics which is of steady behaviour in the surrounding of electrolyte.

The sensor complying with the present invention significantly limits the sources of the residual current.

By using the corundum ceramics a high degree of insulation between the electrodes can be achived and by this way the residual current being caused by insufficient insulation of leakages bridges between the electrodes can be limited.

The residual current caused by diffusion of oxygen from electrolyte can be further eliminated by suitable geometrical arrangement in which the electrolyte is situated inside the body of the probe and covered by massive walls of corundum cermaics.

The desing of the sensor complying with the present invention improves even the dynamic performace characteristics in comparison with the prior-of-the-art enzyme electrodes.

Using corundum ceramics having a very high hardness, a very high quality of the front surface of the sensor can be achieved, and by this way the close application of the membrane system can be ensured. Furthermore, using corundum ceramics enables a defined layer of electrolyte to be made through polishing the sensor surface with a grinding means having a lower hardness than that of the corundum ceramics and having a higher hardness than that of the material of cathode. By this means, the sinking of electrodes in the material of the sensor is carried out. On this sinking, other metals can be polished once more. By this means the thin layers of the electrode system can be definitively prepared having a thickness ranging from 1 to 10 micrometers.

The sensor complying with the objects of the present invention can eliminate significantly the effects of temperature, because the measuring electrodes used for the differential measurement are very near one another, they are isolated by the silver anode (with a very high temperature conductivity) and they are symmetrically arranged. Because of this, the electrodes will operate on the same temperature level as each other and, in the temperature dependence of the sensor, events being influenced by the temperature which are symmetric with reference to the measuring electrodes take no part. As well as the temperature dependence of the electrode process is compensated, the temperature dependence of covering membrane diffusivity and the diffusivity parameters of all membranes being symmetrically arranged in the membrane system for various measuring electrodes, are also compensated. The temperature dependence of the probe, however, is mainly determined by the temperature dependence of the speed of the enzyme reaction which has been used to determine the substance or substances.

The sensor complying with the objects of the present invention decreases the influence of the pressure on the sensor operation considerably by means of the sintered silver anode acting as the support for the membrane system, at the same time, eliminating greater movements of the membrane system in response to exposure to pressure.

The sensor complying with the objects of the present invention solves some of the problems dealing with the immobilization and long term stability of enzymes, and their degradation, since the enzyme membrane, together with the controlling membrane, can be easily replaced and is designed for use for a given number of analyte determinations, thus removing the long term non-stability caused by the degradation of enzyme membranes. Further, the membrane system is made symmetrically, i.e. for any measuring electrode, the membrane system contains membranes having the same parametes as the membrane system of reference and it differs only in the enzyme used.

The sensors complying with the objects of the present invention, further, solve the question of the holder arresting the membrane system, by means of an arresting projection provided preventing a rotation or the application of an undefined membrane system holder to the membranes. The holder made in this way lowers the effects of non-linearity caused by non-homogenous membrane application and, together with the mechanical design it protects the electrode system from stresses occuring in the membrane system, and a free application is realized.

The membrane system of the probe complying with the object of the present invention, one part of which having a covering membrane made of polypropylene or teflon or polyethyleneterephthalate being attached to the sensor body with a tapered coupling, lowers the long term non-stability because the beforementioned membranes possess very low values of diffusivity for molecules of water or substances able poison the cathode.

Further, by means of membrane attachment on the tapered surface, very good sealing of the electrolyte storage reservoir can be attained, as well.

The membrane system of the sensor complying with the object of the present invention lowers by its design even some negative effects of the dynamic behaviour characteristics. By the design of the membrane system and of the electrode system, mechanical stresses are eliminated even in the membrane system. By means of the symmetrical design of both the electrode system and the membrane system mechanical stresses in the membrane system are eliminated, as well. By means of the symmetrical design of the membrane system the effect of both the fluid layer and of deposited substances on the suface of the membrane system can to some extent be eliminated, as well.

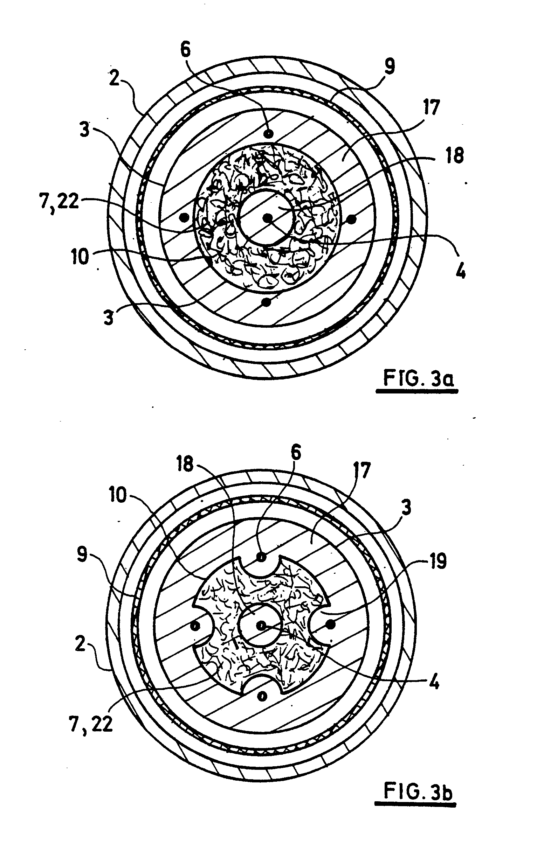

The effect of temperature on the membrane system can be paritally eliminated by means of symmetrical arrangement so that the temperature dependecne of the enzyme may dominate. In case rapid removal of hydroxide ions from cathodes is needed, it is advantageous to modify the front of the sensor as demonstrated in Figure 3.

Some preferred embodiments of the sensor according to the present invention will hereinafter be described with reference to the accompanying schematic drawings in which

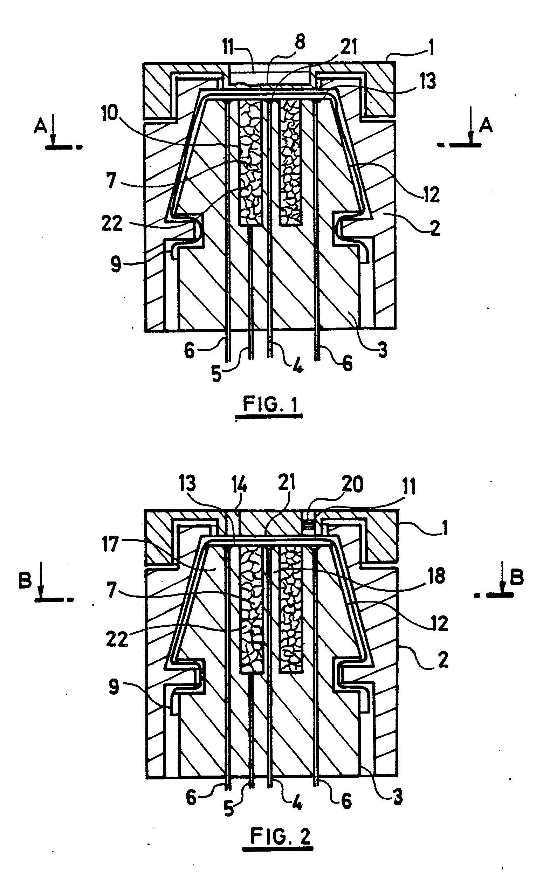

Fig. 1 shows an embodiment fo the sensor in section;

Fig. 2 shows another embodiment thereof in section;

Fig. 3a is a cross-sectional view of the body of sensor together with an electrode system, according to the variant shown in Fig. 1;

Fig. 3b is a cross-sectional view of the body of sensor together with an electrode system, according to the variant shown in Fig. 2;

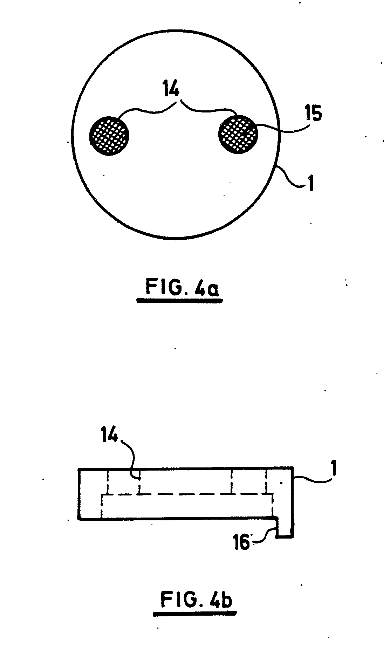

Fig. 4a is a top view of one of the holder embodiments;

Fig. 4b is a side view of the holder embodiment shown in Fig. 4a;

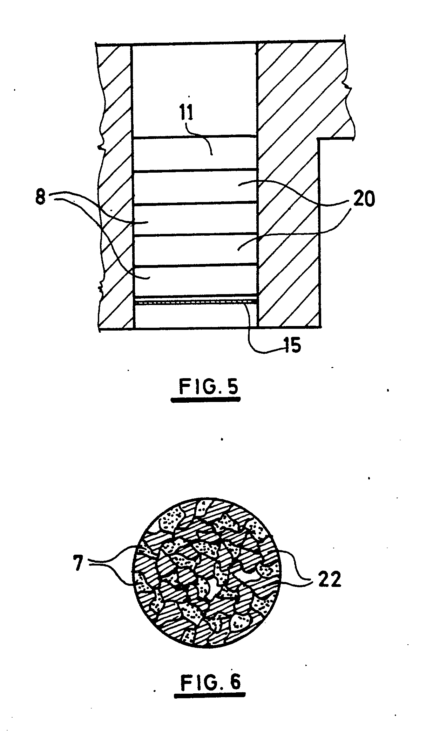

Fig. 5 is a partial sectional view of a membrane system disposed in the holder; and

Fig. 6 is a detail view of a porous current electrode.

The sensor comprises a body 3 terminated by a taper 12, being made of sintered corundum ceramics, the resistivity of which is $10^{12}$ ohms. In it there is the reservoir 10 of electrolyte, arranged having a shape of circular ring (in section) in which the porous silver current electrode 5 is arranged. This is made by sintering silver powder at temperatures ranging from 800 to 900˚C in a hydrogen atmosphere followed by chlorination in gaseous chlorine. In the body 3, further, there are pressed reference platinum electrode 4, its front surface 21 being, e.g., silver plated and chlorinated and, at least one platinum measuring electrode 6. The taper 12 of the body 3 has its front surface polished, having a roughness in the range of from 1 to 10 μm and is covered with the covering membrane 9 which is attached to it with the cover 2 of the sensor. To it the holder 1 is attached, in which ther are arranged both an enzyme membrane 8 and a protective membrane 11.

In a second embodiment, comprises a body 3 which is made of corundum ceramics, the resistivity of which is in excess of $10^{12}$ ohm.m. The body 3 is made as a cylinder terminated with a truncated taper in which ere is created a reservoir 10 having its base in form of circular ring (in section). At the same time, in cross-sectional parallel with the base a first ceramic layer 17 has the shape of a circular ring on inner circumference of which ther are arranged four projections in which there are measuring electrodes 6. The projections 19 project into the space of the reservoir 10 in which there is arranged a porous silver current electrode 5, the pores of which are saturated with an electrolyte, e.g. silver chloride. This electrode surrounds a second layer of ceramics 18 in the shape ofa cylinder in which the platinum reference electrode 4 is present in the front surface 21, which is for example both silver plated and chlorinated. Further, the taper 12 of the body 3 has a front surface 13 which is polished and has a roughness ranging

10

from 0.1 to 25 μm and is covered with a covering membrane 9 being attached to it with a cover 2 made of plastics, the mechanical properties of which ensure an airtight connection of the membrane 9 with the taper 12 of the body 3 of the electrode system. To the cover 2 there is attached a holder 1 being made of plastics, arranged for disassembleable connection.

At the same time, the holder 2 is equipped with an arresting projection with its inner cross-section of circular shape, and in it there are provided openings 14 are secured in which meshes 15 serving as a mechanical reinforcement of the membrane system. The membrane system comprises at least on enzyme membrane 8, soaked with an enzyme, for example, glucose oxidase, over which there is arranged at least one controlling membrane 20, and over it there is at least one protecting membrane 11.

Other design variants of the recoverable enzyme sensor are included in the scope of the present invention.

The operation of the recoverable enzyme sensor is as follows:
The reference electrode 4 is connected to the input of an operation amplifier and picks up the potential in the vicinity of the covering membrane. The current electrode 5 is arranged to carry such electric current that the potential being picked up by the reference electrode 4 may have a predetermined value. The measuring electrode 6, while the suitable potential towards the reference electrode 4 is available, measures the electric current which is proportional to oxygen inflow, e.g., to the space of the electrode system. The concentration of the substance to be determined is proportional to the amount of this substance which is created in the appropriate membrane system. The membrane system consists of plurality of membranes which can be, generally, divided in three groups. The first group comprises enzymatic membranes 8 which catalyse reactions between different chemical substances resulting in the formation of the substance which is then detected by the electrode system, as well. The second group comprises the controlling membrane 20, which limits the flow of the different substances to the enzymatic membranes 8 by diffusion, and thus set the concentration range in which the sensor can operate. The last group comprises the protecting membranes 11 which either cover the membrane system as a whole, or even individiually cover the enzyme membranes 8, and in this way any substances whih could deactivate the enzyme are prevented from entering.

The proper measurement is carried out in such a manner that the substance to be determined is led to the membrane system where it penetrates through the protecting membrane 11 to the controlling membrane 20 in which its flux is controlled towards the enzyme membrane 8 in such a way so that the amount of the product being created by means of the enzyme immobilized in the enzyme membrane 8 may be optimal for the concentration of the substance to be assayed. The product then goes to the next controlling membrane 20 and this process is repeated so long till resulting product is created which is detected by the electrode system.

The utilization of the recoverable enzyme sensor complying with the objects of the present invention has, because of its design, a very broad field of application, especially in biological method. Direct utilization can be found in the following branches: serum manufacture, vaccine preparation, food industry, biochemical procedures, medicine, chemical processes, microbiology, biotransformation control, fermentation process control etc. In the above mentioned branches the application of enzymatic sensors can be expected.

EP 0 372 911 A2

| Item | Designation |
|------|-------------|
| 1 | holder |
| 2 | cover of sensor |
| 3 | body |
| 4 | reference electrode |
| 5 | current electrode |
| 6 | measuring electrode |
| 7 | electrolyte |
| 8 | enzyme membrane |
| 9 | covering membrane |
| 10 | reservoir |
| 11 | protecting membrane |
| 12 | taper |
| 13 | front surface |
| 14 | openings |
| 15 | nettings |
| 16 | arresting projection |
| 17 | first ceramics layer |
| 18 | second ceramics layer |
| 19 | projections |
| 20 | controlling membrane |
| 21 | front surface of reference electrode |
| 22 | grain of sintered silver matter |

## Claims

1. A recoverable enzyme sensor with an interchangeable membrane system characterised in that it comprises a ceramic body 3 having disposed therein a current electrode 5 in the form of a porous metal insert occupying a reservoir 10 for electrolyte, a reference electrode 4 and at least one measuring electrode 6, said electrodes being arranged so that they each have a contact surface at a common surface 13 of the ceramic body such that the contact surface of the current electrode 5 surrounds that of the reference electrode 4 and the contact surface of each measuring electrode 6 is outside the surrounding electrode 5, said common surface 13 being provided with a permeable membrane cover 9, the body 3 being provided with a removable cover 2 to hold an exchangeable enzyme membrane 8 in contact with said common surface 13 covered by the membrane cover 9.

2. The recoverable enzyme sensor of claim 1, in which the body 3 is terminated with a truncated conical taper 12 in which the reservoir 10 is located.

3. The recoverable enzyme sensor according to claim 1 or claim 2 characterized by the body 3 being made of sintered corund ceramics the resistivity of which is at least $10^{13}$ ohm.m.

4. The recoverabe enzyme sensor according to any of claims 1 to 3 characterized by the first ceramics layer 17 of the reservoir 10 being provided with at least one projection 19 arranged on the inner circumference of the circular ring in which projection(s) the measuring electrode(s) 6 are arranged.

5. The recoverable enzyme sensor according to any of claims 2 to 4 characterized by the sensor cover 2 being made of elastomer or tough plastics the mechanical properties of which, e.g. elasticity and toughness, secure the sealed connection of the covering membrane 9 and the taper of the body 3 of the electrode system.

6. The recoverable enzyme sensor according to any of claims 1 to 5 further including a holder 1 being made of plastics for disassembleable connection with the cover 2 of the sensor.

7. The recoverable enzyme sensor according to claim 6 characterized by the holder 1 being provided with an arresting projection 16.

8. The recoverable enzyme sensor according to any of claims 1 to 7 characterized by openings 14 in which the membrane systems are arranged being situated over the different measuring electrodes 6.

9. The recoverable enzyme sensor according to the claim 8 characterized by the holder 1 having a shape of circle and being provided with openings 14 in which the enzyme membrane 8 equipped with the

12

appropriate enzyme, e.g. glucose oxidase, and the controlling membrane 20, the pore area of which corresponds to the size of the substance to be assayed and the protecting membrane 11 the diffuse resistivity of which is negligible are arranged.

10. The recoverable enzyme sensor according to the claim 8 or claim 9 characterized by the openings 14 being provided with meshes.

11. The recoverable enzyme sensor according to any of claims 1 to 10 characterized by the measuring electrode 6 being of metal selected from platinum, silver, gold, tantalum, molybdenum, irridium, osmium or tungsten, or of a non-metal such as carbon.

12. The recoverable enzyme sensor according to any of claims 1 to 10 characterized by the front surface 13 of the taper 12 of the body 3 being provided with the electrode structure created by covering with other metals, selected from gold, silver or platinum or its alloys with the ratio of the gold ranging from 0.1 to 99.9% by weight, with the ratio of the silver ranging from 0.1 to 99.9% by weight and with platinum ratio ranging from 0.1 to 99.9% by weight.

13. The recoverable enzyme sensor according to the claim 12 characterized by the front surface 13 of the taper 12 of the body 3 being polished and having a surface roughness ranging from 0.1 to 50 micrometers.

14. The recoverable enzyme sensor according to claim 13 characterized by the front surface 13 of the taper 12 of the body 3 projecting over measuring electrode 6 by 1 to 10 $\mu$m.

15. The recoverable enzyme sensor according to claim 11 characterized by the measuring electrode 6 being made of an alloy of gold and platinum in the gold ratio 1.0 to 99.9% by weight and platinum ratio 0.1 to 99% by weight.

16. The recoverable enzyme sensor according to any of claims 1 to 15 characterized by the reference electrode 4 being of platinum with the front surface 21 being covered with a layer of silver of palladium.

17. The receoverable enzyme electrode according to any of claims 1 to 15 characterized by the current electrode 5 being made of silver with a purity of at least 97%.

18. The recoverable enzyme sensor according to the claim 17 characterized by the current electrode 5 being made by sintering a silver powder in a hydrogen atmosphere at a temperature ranging from 800 to 900°C, and subsequently being treated by chlorination in gaseous chlorine.

EP 0 372 911 A2

FIG. 1

FIG. 2

Neu eingereicht / Newly filed
Nouvellement déposé

FIG. 3a

FIG. 3b

14

15

1

## FIG. 4a

14

1

16

## FIG. 4b

FIG. 5

FIG. 6